**Europäisches Patentamt**

**European Patent Office**

**Office européen des brevets**

(19)

(11) Publication number : **0 541 330 A2**

(12)

# EUROPEAN PATENT APPLICATION

(21) Application number : **92310055.6**

(22) Date of filing : **03.11.92**

(51) Int. Cl.$^5$ : **C07C 51/00, C07C 59/08**

(30) Priority : **04.11.91 GB 9123354**

(43) Date of publication of application :
**12.05.93 Bulletin 93/19**

(84) Designated Contracting States :
**AT BE CH DE DK ES FR GB IT LI NL SE**

(71) Applicant : **BP Chemicals Limited**
**Belgrave House 76 Buckingham Palace Road**
**London, SW1W 0SU (GB)**

(72) Inventor : **Kelly, Raymond Leslie, BP**
**chemicals Limited**
**Saltend, Hedon**
**Hull, HU12 8DS (GB)**

(74) Representative : **Krishnan, Suryanarayana**
**Kalyana et al**
**BP INTERNATIONAL LIMITED Patents &**
**Agreements Division Chertsey Road**
**Sunbury-on-Thames Middlesex TW16 7LN**
**(GB)**

(54) **Production of hydroxy carboxylic compounds.**

(57)    This invention relates to a process for the production of $\alpha$-hydroxy carboxylic compounds by reacting (a) a carbonyl compound having at least one hydroxyl group $\alpha$- to the carbonyl function with (b) a hydroxy compound of the formula $R^3OH$ where in $R^3$ is an alkyl group, an alkenyl group, a cycloalkyl group or an aryl group. The reaction is carried out at elevated temperatures in the presence of an ionizable transition metal compound supported on an ion-exchange resin as a heterogeneous catalyst. The process is useful for making e.g. lactate esters and lactic acid which are useful items of commerce as solvents and chemical intermediates.

EP 0 541 330 A2

This invention relates to a process for the production of alpha-hydroxy carboxylic compounds from alpha-hydroxy carbonyl compounds by reaction of the latter with an alcohol in the presence of a heterogeneous catalyst.

It is known to produce alpha-hydroxy carboxylic acid salts from alpha-hydroxy carbonyl compounds in the presence of an alkali hydroxide. A specific example of such a process is the production of alkali metal lactates from dihydroxyacetone.

Thus:

$$HO.H_2C.CO.CH_2OH \xrightarrow{\quad NaOH \quad} H_3C.CH(OH).COONa$$

The disadvantage of this process is that the yields are relatively low and that further acidification is required to convert the lactate salts to the corresponding free lactic acid, a very desirable commodity. However, in such a process for each equivalent of lactic acid produced one equivalent of an acid and one equivalent of a base are consumed thus rendering the process commercially non-viable.

It has now been found such hydroxy-carboxylic compounds can be produced by catalytic reaction of an alpha-hydroxy carbonyl compound with an alcohol in the presence of a catalyst.

Accordingly, the present invention is a process for the production of alpha-hydroxy carboxylic compounds said process comprising reacting

(a) a carbonyl compound having at least one hydroxyl group alpha to the carbonyl function with

(b) a hydroxy compound of the formula $R^3OH$ wherein $R^3$ is an alkyl group, an alkenyl group, a cycloalkyl group or an aryl group

at elevated temperature in the presence of an ionisable transition metal compound supported on an ion-exchange resin as a heterogeneous catalyst.

By the term "alpha-hydroxy carboxylic compound" is meant here and throughout the specification that the product of the reaction is primarily lactic acid, an alpha-hydroxy carboxylic acid ester or mixtures thereof. The nature of the product would depend upon the nature of reactant (b).

The carbonyl compounds having at least one hydroxyl group alpha to the carbonyl function and used as reactant (a) in the present invention have the generic formula:

$$HO.H_2C.CO.C(R)(R^1)(R^2) \qquad (I)$$

wherein each of $R$, $R^1$ and $R^2$ may be selected from the groups consisting of hydrogen, hydroxyl, hydroxyalkyl, alkyl, cycloalkyl, alkaryl, aryl, aralkyl, and a sugar residue such that when one of these is a hydroxyl group neither of the other two is a hydroxyl group.

One of the groups $R$, $R^1$ and $R^2$ is most preferably a hydroxyl group. In such a case, the reactant (a) preferably has the generic formula:

$$HOH_2C.CO.C(OH)(R^1)(R^2) \qquad (II)$$

wherein $R^1$ and $R^2$ have the same significance as in formula (I) above.

Where $R$ or $R^1$ or $R^2$:

(i) or all three taken together represent an alkyl group, it is a straight or branched $C_1$-$C_{10}$ alkyl group;

(ii) or all three taken together represent an aryl group, it has one or more aromatic nuclei whether or not forming fused ring structures;

(iii) represent a halo group, it is a chloro or a bromo group;

or,

(iv) represent a sugar residue, such residues being those derived from glucose, fructose or sucrose.

A specific but simple example of compounds of this type is 1,3-dihydroxyacetone represented by $HOH_2C.CO.CH_2OH$ and in the following discussion this compound will hereinafter be denoted as "DHA" and will be used particularly as an example to illustrate the process of the present invention.

As for the reactant (b), it has a generic formula:

$$R^3OH \qquad (III)$$

wherein $R^3$ is H, an alkyl, alkenyl, cycloalkyl or an aryl group.

The group $R^3$ is preferably a $C_1$-$C_{10}$ hydrocarbyl group. Where $R^3$ is a cycloalkyl group, it is preferably a cyclohexyl group.

The reaction is suitably carried out under heterogeneous conditions and under these conditions, it will be understood that the transition metal compound used as catalyst will be supported on an ion-exchange resin.

The catalyst for the reaction is an ionisable transition metal compound derived from a transition metal belonging to any one of Groups III-VIII of the Periodic Table of Elements appearing on pages 448-449 of the Handbook of Chemistry & Physics Ed. by C D Hodgman et al, 44th Edition and published by The Chemical Rubber Publishing Co. (1963). Thus the compound used as catalyst may be for example that of bismuth, aluminium, tin, titanium, zirconium, hafnium, vanadium, niobium, chromium, ruthenium or rhodium. It is preferable that the transition metal compounds used as catalyst are soluble in the reaction system. Thus, the compounds may be selected from halides, nitrates, sulphates, alkoxides, oxides, carboxylates, acetylacetonates and cyclopentadienyl complexes or derivatives thereof of the metals.

The ion-exchange resins used are suitably those which are capable of capturing cations. Such resins may be in the acid form or alkali metal ion-exchanged form. They may be the conventional types or the macroreticular type. Specific examples of such resins include the sulphonated or carboxylated styrene-divinylbenzene and styrene-butadiene resins. Typical examples of such resins include the Amberlyst (Regd Trade Mark) variety of resins. More specifically, Amberlyst-120 is the conventional type and Amberlyst-15 is the macroreticular type.

The amount of transition metal used is suitably from 0.01 to 5 moles, preferably from 0.1 to 2 moles based on the weight of reactant (a).

The reaction is preferably carried out under reflux conditions. Thus the reaction temperature would depend upon the boiling points of the reactants (a) and (b).

The reaction is suitably carried out at elevated pressures.

It is preferable but not essential to remove the water from the reaction system during the reaction when the reactant (b) is an alcohol. Water removal may be achieved by conventional means such as e.g. a Dean and Stark apparatus.

It is preferable to carry out the reaction under an atmosphere inert under the reaction conditions such as e.g. nitrogen.

In some cases, depending upon the nature of reactants (a) and (b) it may be necessary to add a liquid diluent which is inert under the reaction conditions to reduce the viscosity of the reaction system. An example of such a diluent is diethylene glycol dimethyl ether. Such diluents enable the reaction to proceed at a more uniform rate to completion.

The reaction can be carried out batchwise or continuously.

The product of the reaction according to the present invention can be an alpha-hydroxy carboxylic acid or an ester thereof of the formula:

$$R^3OOC.CH(OH).C(R)(R^1)(R^2) \qquad (IV)$$

As is evident from the above formula, when the reaction product is an ester, the group $R^3$ esterifying the carboxylic acid is derived from the alkyl, alkenyl, cycloalkyl or aryl residue of the hydroxylic reactant (b). Thus, the product of the reaction of DHA with, for example, n-butanol in the presence of titanium isopropoxide or titanium lactate catalyst would be n-butyl lactate. In the same reaction, if $R^3 = H$, the reaction product would correspondingly be lactic acid.

The process of the present invention is particularly suited to producing lactic acid and lactate esters.

Lactic acid and lactate esters are items of commerce in their own right for uses such as solvents and as chemical intermediates. The esters can, however, also be hydrolysed to lactic acid by conventional means.

The present inventions is further illustrated with reference to the following Examples illustrated using DHA.

Examples

RESIN PREPARATIVE PROCEDURES

Note: Dihydroxyacetone is commercially available as the dimer which is readily dissociated in solution to the monomeric form. Diethylene glycol dimethyl ether was present as an internal standard for analysis by gas chromatography. Tilcom TL is an aqueous solution of titanium lactate and a sample was provided by Tioxide for evaluation.

1) CHROMIUM (GEL) RESIN

400g of the sodium form of Amberlite IR-120(plus) (a gel-type ion-exchange resin) was washed thoroughly with deionised water and then stirred for 24hr with 200g of hydrated chromium (III) sulphate dissolved in about 1000g water. It was then stirred three times more with fresh solution of the same composition for 24 hours each time. It was then packed into a tube and 250g hydrated chromium (III) sulphate dissolved in 2000g water was slowly passed through it over a period of 96 hours. It was then washed thoroughly by stirring with boiling deionised water for several hours. Analysis: 6.81% chromium, 0.028% sodium w/w in an oven-dried sample.

2) CHROMIUM (MACRORETICULAR) RESIN

A chromium-loaded resin was prepared using the sodium form of a macroreticular resin Amberlyst 15, obtained by stirring the acid form with excess aqueous sodium hydroxide for several hours followed by thorough washing with water before stirring twice for several hours each time with 12.5g of hydrated chromium(III) sulphate dissolved in 100g water. Analysis: 6.24% chromium, 0.13% sodium w/w in an oven-dried sample.

3) TIN RESIN

A tin-loaded resin was prepared using 430g of the sodium form of amberlite IR-120(plus). 730g of anhydrous tin(II) chloride was dissolved in 3750g water, with 220ml conc hydrochloric acid added to redissolve the hydrolysed tin precipitate. This solution was slowly pumped through the resin over a period of 72hr. The resin was then washed thoroughly with deionised water and boiled in more deionised water for several days. Analysis: 18.3% tin, 0.061% sodium w/w in any over-dried sample.

4) ZIRCONIUM RESIN

A zirconium-loaded resin was prepared as in procedure 2 above, but using amberlite IR-120(plus) as the resin and with each portion of chromium sulphate replaced by 10g of zirconyl chloride octahydrate in sufficient water to dissolve it totally (approx 350ml). Each contact period was 24hr. It was then washed thoroughly in deionised water and boiled for several hours in deionised water.

5) VANADIUM RESIN

A vanadium-loaded resin was prepared as in procedure 4 above, but with each portion of zirconyl chloride replaced by 10g of 50% aqueous vanadyl chloride solution in 100g water.

6) ALUMINIUM RESIN

An aluminium-loaded resin was prepared as in procedure 4 above, but with each portion of zirconyl chloride replaced by 10g of aluminium sulphate dissolved with heating in 150g water.

7) TITANIUM RESIN

A titanium-loaded resin was prepared as in procedure 4 above, but with each portion of zirconyl chloride replaced by 30g of titanium lactate solution (Tilcom TL - supplied by Tioxide plc).

8) BISMUTH RESIN

A bismuth-loaded resin was prepared as in procedure 4 above, but with each portion of zirconyl chloride solution replaced by a solution of 10g of bismuth(III) chloride in 40ml water, with addition of concentrated hydrochloric acid until the hydrolysed bismuth precipitate redissolved before the resin was added. The solution was replaced daily for 5 days. Analysis: 0.3% bismuth, 0.072% sodium w/w in an oven-dried sample.

Example 1

DHA-CHROMIUM RESIN ONLY

0.30g dihydroxyacetone (DHA) in 1.70g water was heated to 78°C for 24hr with 3.89g chromium-loaded resin prepared as in procedure 1. Conversion of DHA was 100%; selectivity to lactic acid was 57% and to pyruvic aldehyde 2%. On repeating the procedure with the same portion of resin, the selectivities altered to 86% and 1% respectively, and on further repetition, 94% and 0% respectively. Examples 2-9 using the following resins:

DHA- Sn/Zr/V/Ti/Al/Bi/Cr-RESINS

The tin-, zirconium-, vanadium-, titanium-, aluminium-, bismuth and chromium- loaded gel resins prepared in procedures 3,4,5,7,6,8 and 1 respectively and the chromium-loaded macroreticular resin prepared in procedure 2 was flooded with 40% aqueous DHA solution to the top of the resin layer and heated to 100°C for 24hr. This was twice repeated using the same portions of resin in order to load them fully with absorbed lactate. Subsequently, the same process was carried out but with samples taken after 2, 5 and 24 hours. Conversions of DHA (C) and selectivity to lactic acid (S) were found to be:

| Example | Metal | ..2hr.. | | ..5hr.. | | ..24hr.. | |
|---|---|---|---|---|---|---|---|
| | | C | S | C | S | C | S |
| 2 | tin | 98 | 53 | 99 | 68 | 100 | 70 |
| 3 | zirconium | 91 | 13 | 99 | 20 | 100 | 45 |
| 4 | vanadium | 81 | 13 | 88 | 26 | 100 | 40 |
| 5 | titanium | 85 | 4 | 100 | 5 | 100 | 10 |
| 6 | aluminium | 69 | 14 | 100 | 42 | 100 | 58 |
| 7 | bismuth | 99 | 11 | 100 | 18 | 100 | 39 |
| 8 | chromium(gel) | 98 | 54 | 100 | 87 | 100 | 86 |
| 9 | chromium(macroreticular) | 81 | 38 | 96 | 52 | 100 | 65 |

C - Conversion

S - Selectivity

**Claims**

1. A process for the production of $\alpha$-hydroxy carboxylic compounds, said process comprising reacting:
   (a) a carbonyl compound at least one hydroxyl group $\alpha$- to the carbonyl function with
   (b) a hydroxy compound of the formula $R^3OH$ wherein $R^3$ is an alkyl group, an alkenyl group, a cycloalkyl group or an aryl group at elevated temperature in the presence of a ionizable transition metal compound supported on an ion-exchange resin as a heterogeneous catalyst.

2. A process according to Claim 1 wherein reactant (a) has the generic formula:
$$HO.H^2C.CO.C(R)(R^1)(R^2) \qquad (I)$$
wherein each of R, $R^1$ and $R^2$ may be selected from the group consisting of hydrogen, hydroxyl, hydroxyalkyl, alkyl, cycloalkyl, alkaryl, aryl, aralkyl and a sugar residue such that when one of these is a hydroxyl group neither of the other two is a hydroxyl group.

3. A process according to Claim 1 or 2 wherein at least one of the groups R, $R^1$ and $R^2$ is a hydroxyl group.

4. A process according to Claim 2 wherein if R or $R^1$ or $R^2$:
   (i) or all three taken together represent an alkyl group, it is a straight or branched chain $C_1$-$C_{10}$ alkyl group;
   (ii) or all three taken together represent an alkyl group, it has one or more aromatic nuclei whether or not forming fused ring structures;
   (iii) represent a halo group, it is a chloro or a bromo group; or
   (iv) represent a sugar residue, such residues are those derived from glucose, fructose or sucrose.

5. A process according to any one of the preceding Claims wherein the reactant (a) is dihydroxyacetone.

6. A process according to Claim 2 wherein in reactant (b) $R^3$ is H, an alkyl, alkenyl, cycloalkyl or an aryl group.

7. A process according to Claim 6 wherein $R^3$ is a $C_1$-$C_{10}$ hydrocarbyl group.

8. A process according to any one of the preceding Claims wherein the ionizable transition metal compound is derived from a transition metal belonging to any one of Groups II-VIII of the Periodic Table of Elements appearing on pages 448-449 of the Handbook of Chemistry & Physics, Ed C D Hodgman et al, 44th Edition and published by The Chemical Rubber Publishing Co (1963).

9. A process according to any one of the preceding Claims wherein the ionizable transition metal compound is derived from one or more of the following transition metals: bismuth, aluminium, tin, titanium, hafnium, vanadium, niobium, chromium, ruthenium and rhodium.

10. A process according to any one of the preceding Claims wherein the ionzable transition metal compounds are selected from the halides, nitrates, sulphates, alkoxides, carboxylates, acetylacetonates and cyclopentaidenyl complexes.

11. A process according to any one Claims wherein the ion exchange resin used as support is capable of capturing cations in the transition metal compound.

12. A process according to Claim 11 wherein the ion exchange resin is selected from sulphonated or carboxylated (i) styrene-divinyl benzene and (ii) styrene-butadiene resins.

13. A process according to any one of the preceding Claims wherein the amount of transition metal in the catalyst used is from 0.01 to 5 moles based on the weight of reactant (a).

14. A process according to ant one of the preceding Claims wherein the $\alpha$-hydroxy carboxylic compound formed has the formula:
$$R^3OOC.CH(OH).C(R)(R^1)(R^2) \qquad (IV)$$
wherein the notations R, $R^1$, $R^2$ and $R^3$ have the same significance as in Claims 1 and 2.

15. A process according to any one of the preceding Claims wherein diydroxyacetone is reacted with n-butanol to form n-butyl lactate and/or lactic acid.